(19) **Europäisches Patentamt**
**European Patent Office**
**Office européen des brevets**

(11) **EP 2 810 042 B1**

(12) **FASCICULE DE BREVET EUROPEEN**

(45) Date de publication et mention
de la délivrance du brevet:
**31.10.2018   Bulletin 2018/44**

(21) Numéro de dépôt: **13704728.8**

(22) Date de dépôt: **29.01.2013**

(51) Int Cl.:
*G01N 1/38* *(2006.01)*        *G01N 35/00* *(2006.01)*
*G01N 35/10* *(2006.01)*

(86) Numéro de dépôt international:
**PCT/EP2013/051619**

(87) Numéro de publication internationale:
**WO 2013/113670 (08.08.2013 Gazette 2013/32)**

(54) **DISPOSITIF ET PROCEDE POUR EFFECTUER DES MESURES HEMATOLOGIQUES ET BIOCHIMIQUES A PARTIR D'UN ECHANTILLON BIOLOGIQUE**

VORRICHTUNG UND VERFAHREN ZUR DURCHFÜHRUNG HÄMATOLOGISCHER UND BIOCHEMISCHER MESSUNGEN AUS EINER BIOLOGISCHEN PROBE

DEVICE AND METHOD FOR CARRYING OUT HAEMATOLOGICAL AND BIOCHEMICAL MEASUREMENTS FROM A BIOLOGICAL SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité:  **02.02.2012   FR 1250961**

(43) Date de publication de la demande:
**10.12.2014   Bulletin 2014/50**

(73) Titulaires:
• **Horiba ABX SAS**
  **34184 Montpellier (FR)**
• **Bibette, Jérôme**
  **75005 Paris (FR)**

(72) Inventeurs:
• **BIBETTE, Jérôme**
  **F-75005 Paris (FR)**

• **NERIN, Philippe**
  **34820 ASSAS (FR)**
• **GINEYS, Jean-Philippe**
  **F-30440 Roquedur (FR)**
• **CAUET, Gilles**
  **F-34270 Fontanès (FR)**

(74) Mandataire: **IPAZ**
  **18 rue de la République**
  **34000 Montpellier (FR)**

(56) Documents cités:
**EP-A2- 0 409 606          WO-A2-02/37078**
**US-A- 4 030 888          US-A- 5 183 638**
**US-A- 5 215 714          US-A- 5 290 708**
**US-A- 5 939 326          US-A- 6 106 778**
**US-A- 6 159 740          US-A1- 2009 117 620**

**EP 2 810 042 B1**

**Description**

**Domaine technique**

La présente invention concerne un dispositif et un procédé d'analyse permettant d'effectuer des mesures biologiques, notamment hématologiques et biochimiques, à partir d'un échantillon biologique, notamment un prélèvement de sang total.
Le domaine de l'invention est plus particulièrement mais de manière non limitative celui des systèmes d'analyse.

**Etat de la technique antérieure**

La différenciation et le comptage d'éléments cellulaires du sang, complétés par le dosage d'au moins un analyte sanguin, présentent un intérêt important dans le domaine du diagnostic. En effet, la plupart des demandes d'analyses biologiques concernent la numération formule sanguine (NFS) en plus de laquelle on demande le dosage d'un ou plusieurs analytes tels que la Protéine C-réactive (CRP) ou encore la Pro-Calcitonine (PCT).
A l'heure actuelle, ces analyses requièrent deux prélèvements : un premier prélèvement réalisé sur anticoagulant, destiné à l'analyse hématologique et un second prélèvement, sans anticoagulant, destiné à l'analyse biochimique.
L'échantillon destiné à l'analyse biochimique est centrifugé avant d'être traité dans les analyseurs destinés au dosage d'une biomolécule sérique ou plasmatique. L'échantillon destiné à l'analyse hématologique est agité afin de maintenir les cellules en suspension dans le tube avant d'être traité par les automates d'hématologie. De plus, et bien souvent, les deux prélèvements sont destinés à des équipements distincts situés dans des laboratoires différents. Ce processus est généralement long et couteux. Or, nombre de situations requièrent une mesure rapide de la numération formule sanguine et le dosage d'un ou plusieurs analytes. Cette approche est envisageable dès lors où la technologie permet le dosage sur sang total (c'est-à-dire le plasma avec les cellules sanguines). Si cette rapidité apporte un bénéfice incontestable au patient, elle contribue aussi à réduire les délais et les coûts de prise en charge.
Par exemple, ce type d'analyse est requis en médecine ambulatoire, en médecine d'urgence ou encore dans des laboratoires de routine où la cadence d'analyse est un facteur déterminant. Dans toutes ces situations, on cherche à simplifier les opérations dites pré-analytiques, nécessaires à la préparation de l'échantillon avant l'analyse, et on cherche une grande rapidité d'analyse avec des niveaux de précision et de reproductibilité élevés pour permettre un diagnostic fiable.
Le couplage d'une mesure hématologique et d'un essai biologique est par exemple décrit dans le document US 6 106 778 de Oku et al. Ce document décrit un dispositif d'analyse comprenant un module d'hématologie et un module de biochimie, et permettant la mesure des paramètres hématologiques suivants : nombre de globules blancs (WBC count), nombre de globules rouges (RBC count), nombre de plaquettes (PLT count), volume globulaire moyen (MCV), hématocrite (Hct) et hémoglobine (Hgb).
Un inconvénient majeur de ce dispositif est qu'il ne permet pas des cadences d'analyses élevées. En effet, un seul chariot et une seule aiguille de prélèvement sont utilisés pour le prélèvement et la distribution du sang et des réactifs au niveau du module d'hématologie et du module de biochimie. Il s'ensuit que le taux d'occupation de l'aiguille est trop élevé pour permettre des temps d'analyse hématologique inférieurs par exemple à 60 secondes.
Un autre inconvénient provient du fait qu'une même aiguille est utilisée pour stocker l'échantillon de sang et les réactifs avant l'étape d'hémodilution, ce qui pose un problème de contamination (carry over) préjudiciable aux dosages sensibles d'analytes faiblement abondants.
Enfin, l'essai biologique tel que décrit dans ce document ne permet pas d'atteindre une vitesse de réaction immunologique pour le dosage de l'analyte recherché qui soit compatible avec des cadences d'analyse élevées tout en conférant une mesure sensible, reproductible et sans contamination inter-échantillon. Il est basé sur un principe bien connu faisant usage des phénomènes d'agrégation colloïdale. Ce test, souvent appelé « LAI » pour « latex agglutination immunoassay » (en Anglais) est basé sur le principe suivant : des anticorps spécifiques d'un antigène donné sont greffés sur des particules colloïdales, ainsi la capture de l'antigène par au moins deux particules conduit à un agrégat qui modifie la turbidité de la solution, ce qui autorise un dosage quantitatif des antigènes.
La vitesse de cette réaction d'agrégation dépend bien entendu en partie de la concentration en antigènes. Lorsqu'on cherche la meilleure sensibilité, la concentration en particules doit toujours excéder celle en antigènes. En particulier, une concentration en particules de l'ordre de 10 fois celle des antigènes assure le meilleur compromis sachant que si le nombre de doublets attendus (fixé par le nombre d'antigènes) est très minoritaire, comparé au nombre de singulets (particules non agrégées), le rapport signal sur bruit en est affecté.
La vitesse de la réaction d'agrégation colloïdale est aussi liée aux coefficients de diffusion des particules (translation et rotation) ainsi qu'à la concentration surfacique en anticorps greffés sur la surface des particules colloïdales. Il est aussi admis que cette vitesse de réaction impose pour un temps d'incubation donné, la sensibilité et le seuil de détection du test. Ainsi, un temps d'incubation plus court nécessitera, pour une sensibilité égale, une vitesse d'agrégation plus importante.

Plusieurs approches ont été proposées pour accélérer cette réaction sans augmenter significativement le bruit non spécifique, et donc offrir soit une plus grande sensibilité, soit une plus forte cadence d'essai, en réduisant le temps d'incubation. Ces méthodes ont toutes en commun d'augmenter la fréquence de collision des particules en induisant une augmentation locale pendant l'application d'un champ extérieur, de la concentration en particules colloïdales.

Trois approches distinctes sont connues à ce jour : l'utilisation d'ondes stationnaires ultrasonores, l'utilisation de champs électriques alternatifs dans des systèmes bidimensionnels ou tridimensionnels macroscopiques, et enfin l'utilisation de champs magnétiques associés à des particules colloïdales super paramagnétiques. Dans le cas des ondes ultrasonores stationnaires, l'enrichissement local en particules intervient dans les zones de forte pression acoustique ; dans le cas des systèmes bidimensionnels confinés avec application d'un champ alternatif électrique haute fréquence dans le sens perpendiculaire, l'enrichissement local intervient via l'existence de forces attractives d'origine hydrodynamique (induite par la circulation des ions) ; dans le cas des systèmes macroscopiques avec application d'un champ alternatif électrique haute fréquence, l'enrichissement local intervient sous forme de chaînes de particules sous l'action des forces colloïdales dipolaires (induites par la différence de polarisabilité) ; dans le cas de systèmes macroscopiques avec particules colloïdales superparamagnétiques soumis à un champ magnétique homogène, l'enrichissement intervient sous forme de chaînes induites par les interactions dipolaires magnétiques.

On connaît notamment le document EP 1 446 666 de Bibette et al. qui décrit une méthode pour détecter des analytes en utilisant des particules magnétiques colloïdales, c'est-à-dire des particules magnétiques avec une taille comprise entre 5 nanomètres et 10 micromètres.

Ces particules sont fonctionnalisées à leur surface avec un ligand qui peut être un anticorps, un antigène ou toute autre molécule capable de lier spécifiquement l'analyte à doser et incorporent un matériau magnétique apte à adopter un comportement superparamagnétique. Sous l'effet de l'application d'un champ magnétique, elles ont tendance à s'agglutiner en chaîne, favorisant ainsi l'accrochage de l'analyte à deux particules distinctes. Après annulation du champ magnétique, seules les particules fixées par l'analyte demeurent organisées en chaînes permanentes. La mesure est effectuée par une méthode optique, par microscopie ou mesure de densité. Les exemples de mesure décrits portent notamment sur des dosages d'analytes dans une solution de plasma d'étalonnage.

Un but de la présente invention est de proposer un dispositif et un procédé pour effectuer des mesures hématologiques et biochimiques à partir d'un échantillon de sang total, c'est-à-dire dans lequel les cellules n'ont pas été retirées au préalable, de manière rapide et fiable.

Un autre but de la présente invention est de proposer un dispositif et un procédé pour effectuer des mesures hématologiques et biochimiques à partir d'un seul prélèvement de sang total de manière automatisée.

Un autre but de la présente invention est de proposer un dispositif et un procédé pour effectuer des mesures hématologiques et biochimiques à cadence élevée à partir d'échantillons de sang total, de l'ordre par exemple d'une mesure par minute.

Un autre but de la présente invention est de proposer un dispositif et un procédé pour effectuer des mesures hématologiques et biochimiques à partir d'un échantillon de sang total permettant d'atteindre une sensibilité et une dynamique de mesure élevées pour les mesures biochimiques.

Un but de la présente invention est enfin de proposer un dispositif et un procédé pour effectuer des mesures hématologiques et biochimiques à partir d'un échantillon de sang total dans lequel les risques de contamination croisée entre l'échantillon de sang et les réactifs sont minimisés.

### Exposé de l'invention

Cet objectif est atteint avec un dispositif d'analyse de paramètres biologiques à partir d'un échantillon biologique, tel que défini dans la revendication 1.

Suivant des modes de mise en oeuvre :

- Les seconds moyens de préparation peuvent être aptes à effectuer au moins une opération de lyse ;
- Le premier échantillon issu des premiers moyens de préparation peut avoir au préalable subi une opération de lyse ;
- L'opération de lyse peut être effectuée avec un réactif lytique, ou par d'autres moyens tels qu'une méthode physique ;
- Les seconds moyens de transfert peuvent être partiellement ou totalement distincts des premiers moyens de transfert. Ils peuvent être aptes à fonctionner de manière simultanée ou en parallèle avec les premiers moyens de transfert ;

Les particules fonctionnalisées peuvent comprendre :

- des particules comprenant un matériau ferromagnétique ;
- des particules pourvues d'un noyau à forte teneur en oxyde de fer entouré d'une coque en matériau polymère ;
- des particules de forme essentiellement sphérique d'un diamètre moyen inférieur à 1 micromètre, de préférence inférieur à 500 nanomètres, et encore de préférence compris entre 100 et 300 nanomètres.

Les moyens de mesure d'immuno-détection peuvent comprendre des moyens de mesure optique avec au moins une source de lumière et au moins un détecteur placés à proximité de la cuve de mesure, laquelle étant pourvue, au moins au niveau desdits moyens de mesure optique, de parois sensiblement transparentes.

Les moyens de mesure d'immuno-détection peuvent comprendre en outre des moyens de conditionnement optiques aptes à produire à partir d'au moins une source de lumière un faisceau de lumière collimaté traversant la cuve de mesure.

Le dispositif selon l'invention peut comprendre au moins une source de lumière apte à émettre dans des longueurs d'ondes optiques comprises entre 400 nanomètres et 4 micromètres, et de préférence comprises entre 600 nanomètres et 900 nanomètres.

Suivant des modes de réalisation, le dispositif selon l'invention peut comprendre en outre :

- un électroaimant apte à produire un champ magnétique dans la cuve de mesure,
- des moyens de régulation de la température de la cuve de mesure, et/ou des moyens de mesure de la température de la cuve de mesure,
- un conteneur de stockage régulé à une température optimale pour stocker le réactif de dosage, laquelle température pouvant être de préférence comprise entre 5°C et 15°C, et
- des moyens d'agitation pour mettre et/ou maintenir en suspension les particules fonctionnalisées dans le réactif de dosage stocké, lesquels pouvant être par ultrasons et comprendre au moins l'un des moyens suivants : une sonotrode externe couplée au conteneur de stockage, une sonotrode immergée dans le réactif de dosage.

Suivant des modes de réalisation, les premiers moyens de transfert peuvent comprendre une aiguille de prélèvement et des moyens de déplacement de ladite aiguille de prélèvement.

Suivant des modes de réalisation, les seconds moyens de transfert peuvent comprendre :

- une aiguille de prélèvement et des moyens de déplacement de ladite aiguille de prélèvement aptes à transférer du réactif de dosage dans la cuve de mesure,
- une vanne d'échantillonnage apte à prélever un échantillon dans les premiers moyens de préparation.

L'échantillon biologique peut comprendre tout type d'échantillon biologique adapté, tel que par exemple un échantillon de moelle osseuse, de liquide céphalorachidien, de lymphe, d'urine, ou un échantillon de sang total....

Ainsi, suivant des modes de mise en oeuvre particuliers, le dispositif selon l'invention peut être un dispositif pour d'analyse de paramètres biologiques à partir d'un échantillon biologique comprenant un échantillon de sang total.

Il peut comprendre en outre des moyens de mesure de composante cellulaire aptes à fournir au moins une mesure d'hématocrite.

Suivant des modes de réalisation, cette mesure d'hématocrite peut être fournie :

- à partir de l'échantillon biologique,
- à partir d'un second échantillon issu des premiers moyens de préparation.

Plus généralement, les moyens de mesure de composante cellulaire peuvent comprendre des moyens de mesure d'hématocrite, ou des moyens de mesure d'hématologie aptes à fournir au moins une mesure d'hématologie parmi une différenciation et/ou un comptage d'éléments du sang, un dosage de l'hémoglobine, une mesure de l'hématocrite, une mesure de volume de cellules.

Suivant un autre aspect de l'invention, il est proposé un procédé d'analyse de paramètres biologiques à partir d'un échantillon biologique, tel que défini dans la revendication 17.

Le procédé selon l'invention peut en outre comprendre une étape de lyse de l'échantillon préalablement au dosage de l'analyte d'intérêt. Il peut en particulier comprendre une étape de réalisation par les seconds moyens de préparation, sur le premier échantillon issu des premiers moyens de préparation, d'au moins une opération de lyse. Cette opération de lyse peut être effectuée avec un réactif lytique.

Le dosage d'au moins un analyte d'intérêt peut comprendre des étapes de :

- introduction dans la cuve de mesure d'une solution de mesure comprenant au moins un échantillon et du réactif de dosage,
- mesure d'une première intensité optique au travers de la cuve de mesure,
- application d'un champ magnétique dans la cuve de mesure pendant une durée déterminée, de telle sorte à permettre l'agrégation des particules fonctionnalisées sous l'effet du champ magnétique,

- après l'arrêt du champ magnétique, mesure d'une seconde intensité optique représentative de l'agrégation résiduelle des particules fonctionnalisées due aux couplages entre ligand et analyte d'intérêt,
- calcul d'une variation de densité optique en fonction du ratio desdites premières et secondes intensités optiques, et
- application d'une fonction d'étalonnage préalablement déterminée pour calculer la concentration de la protéine d'intérêt à partir de la variation de densité optique.

Suivant des modes de mise en oeuvre, l'échantillon biologique peut être dilué à un taux supérieur à x500 dans la solution de mesure.

Suivant des modes de mise en oeuvre, le procédé selon l'invention peut être un procédé pour d'analyse de paramètres biologiques à partir d'un échantillon biologique qui comprend un échantillon de sang total.

Il peut mettre en oeuvre pour le dosage de l'analyte d'intérêt :

- un réactif lytique comprenant de la saponine,
- une solution tampon apte à maintenir un pH optimal.

Suivant des modes de mise en oeuvre :

- l'analyte d'intérêt peut être une protéine d'intérêt,
- le procédé selon l'invention peut mettre en oeuvre des particules fonctionnalisées avec un ligand apte à permettre un dosage de la protéine C-réactive (CRP).

Avantageusement, la mise en oeuvre de particules colloïdales avec des propriétés superparamagnétiques et d'un champ magnétique homogène permet un bon contrôle et d'excellentes performances en termes de cinétique de réaction. La principale raison est liée à l'origine des forces en jeu : le caractère superparamagnétique (susceptibilité relative de l'ordre de 1) des colloïdes permet d'appliquer des forces de l'ordre de quelques dizaines de piconewtons sur des colloïdes de 200 nm de diamètre environ, sans bloquer la diffusivité rotationnelle. Un contact intime entre particules dans la chaîne peut s'établir tout en laissant les particules soumises à l'action du mouvement Brownien rotationnel, et les particules voisines ayant capturé un antigène (ou un analyte ou une protéine d'intérêt) peuvent trouver rapidement l'orientation propice à former les doublets. Ainsi, en quelques secondes, un antigène ou une protéine d'intérêt peut être dosé avec une sensibilité de l'ordre de la pico mole par litre.

On rappelle que les propriétés superparamagnétiques se traduisent par le fait que l'aimantation des particules paraît nulle en l'absence de champ magnétique, mais qu'elles présentent une susceptibilité magnétique élevée en présence de ce champ. Elles sont naturellement dispersées en l'absence de champ magnétique et elles se regroupent sous forme de chaînes en présence de ce dernier.

L'efficacité et la rapidité de la réaction d'agrégation permettent d'obtenir des cadences de mesure supérieures aux dispositifs de l'art antérieur car la reconnaissance entre un anticorps (ou un ligand) et un antigène (ou un analyte ou une protéine d'intérêt) est ici forcée par le champ magnétique et non laissée au hasard de rencontre qui résulterait du choc aléatoire des particules initié par le mouvement Brownien. Cette rencontre forcée par le champ magnétique permet de former rapidement des agrégats et donc, dans un temps donné, d'augmenter considérablement la sensibilité d'un essai biologique.

Cette efficacité permet en outre d'utiliser des taux de dilution élevés (supérieurs à x500, voire x1000 ou x1500) tout en conservant des temps de mesure raisonnables.

Dans le domaine des analyses sanguines en particulier, la possibilité d'utiliser des taux de dilution élevés pour le dosage des analytes d'intérêt ou protéines sur sang total est un aspect particulièrement avantageux de l'invention. Dans les dispositifs de l'art antérieur qui utilisent une méthode d'agrégation de particules de latex par agitation simple, tel que par exemple celui décrit dans US 6 106 778, du fait de la faible cinétique de réaction, le taux de dilution utilisé est plutôt de l'ordre de x15 à x51 (par exemple pour le Micros CRP200®). Or le milieu sanguin avec l'hémoglobine est très absorbant dans les longueurs d'onde visibles. Cela oblige à faire des mesures optiques dans l'infrarouge, et du fait des grandes longueurs d'onde, ces mesures sont de simples mesures d'absorption ou de densité optique influencées par tous les effets d'absorption et de turbidité du milieu.

Avec des taux de dilution de l'ordre de x500 ou plus, le milieu analysé est nettement moins absorbant pour les longueurs d'onde visibles. Cela permet de mettre en oeuvre des longueurs d'onde plus courtes, et d'obtenir une mesure dans laquelle les pertes dues au phénomène de diffusion sur les agrégats de particules sont importantes relativement à l'absorption du milieu. La mesure optique devient ainsi beaucoup plus (ou mieux) représentative du phénomène recherché et permet d'obtenir des plages de sensibilité très élevées et un niveau de bruit de mesure plus faible que les simples mesures d'absorption de l'art antérieur.

En outre, l'oxyde de fer utilisé pour les particules colloïdales possède des propriétés d'absorption en fonction de la longueur d'onde optique proches de celles de l'hémoglobine. Tout comme pour l'hémoglobine, il existe des longueurs d'onde de transparence situées entre 600 et 900 nm où la mesure peut s'effectuer sans trop de gêne par rapport aux propriétés d'absorption du milieu. Ainsi, les particules non agrégées perturbent peu les propriétés spectroscopiques du milieu et donc la mesure. On diminue l'effet de matrice, c'est-à-dire l'influence du milieu sur les éléments à doser.

A titre d'exemple, le dispositif selon l'invention permet un dosage de la protéine C-Réactive (CRP) dans la plage 2-200 mg/L en utilisant un taux de dilution de l'ordre de x1500.

Suivant un autre aspect avantageux de l'invention, l'aiguille de prélèvement qui sert à transférer le réactif de dosage dans la cuve de mesure n'est jamais en contact avec l'échantillon de sang total non dilué. Cela représente un réel avantage par rapport à l'art antérieur car cette disposition permet, d'une part, de limiter la pollution du réactif de dosage par l'échantillon de sang lui-même, d'autre part, de réduire l'effet d'interférence « carry over » d'un échantillon à l'autre sur la dilution finale.

Suivant encore un aspect avantageux, les mesures de composante cellulaire (ou mesures d'hématologie) et d'immuno-détection sont effectuées en parallèle, et permettent d'obtenir des cadences de mesure élevées, de l'ordre d'une minute pour un échantillon de sang.

En outre, le couplage des mesures d'hématologie et d'immuno-détection ne se limite pas à la partie fluidique ou à l'échantillonnage. En effet les mesures d'immuno-détection nécessitent des calibrations qui peuvent avantageusement exploiter des paramètres issus des mesures d'hématologie.

Par exemple, la valeur de l'hématocrite peut être utilisée pour exprimer la concentration de l'analyte (initialement dosé sur sang total) dans la fraction sérique de l'échantillon : dans ce cas on divise la concentration de l'analyte dosé par la valeur de l'hématocrite qui est la somme du leucocrite, du thrombocrite et de l'érythrocrite. Il est encore possible de diviser la valeur obtenue sur sang total par un polynôme de degré supérieur ou égal à 2 pour prendre en compte des effets non linéaire introduits par le dispositif de mesure.

Selon un autre exemple, on peut prendre en compte le comptage différentiel des cellules pour détecter certaines anomalies sources d'interférences, telles qu'un nombre trop grand de globules rouges et/ou de globules blancs et/ou de plaquettes. Dans ce cas l'affichage du résultat du dosage de l'analyte n'est pas affiché.

On peut également prendre en compte ces mesures de comptage pour corriger la mesure de la densité optique obtenue lors des mesures de dosage, par exemple en appliquant une relation de correction polynomiale telle que :

$$DO = DO° - \sum_i a_i (GR\#)^i - \sum_j b_i (GB\#)^j - \sum_k c_i (PLT\#)^k$$

où :

i,j,k sont des entiers ;
GR#, GB# et PLT# sont les comptes des globules rouges, des globules blancs et de plaquettes exprimés par unité de volume (L) ;
$a_i$, $b_i$, $c_i$ sont des coefficients ;
DO° est la valeur de la densité optique telle que mesurée, non corrigée et comportant des artefacts de mesure lié à un excès de cellules ou débris correspondant dans le milieu réactionnel ;
DO est la valeur de la densité optique corrigée à partir des données issues des mesures d'hématologie.

Cette relation n'est pas limitative, elle peut notamment faire intervenir d'autres paramètres tels que la volumétrie cellulaire.

## Description des figures et modes de réalisation

D'autres avantages et particularités de l'invention apparaîtront à la lecture de la description détaillée de mises en oeuvre et de modes de réalisation nullement limitatifs, et des dessins annexés suivants :

- la figure 1 présente un schéma fonctionnel d'un dispositif selon l'invention, selon un premier mode de réalisation,
- la figure 2 présente une représentation schématique d'un dispositif selon l'invention, selon un premier mode de réalisation,
- la figure 3 présente une vue en coupe de la cuve de mesure d'immuno-détection,
- la figure 4 présente un exemple d'acquisition de signaux de mesure lors des mesures de dosage, avec à la figure 4(a) le champ magnétique et à la figure 4(b) le signal optique,
- la figure 5 présente un exemple de courbe de calibration de dosage de la protéine C-réactive dans un échantillon de sang total,
- la figure 6 illustre le fonctionnement de la vanne d'échantillonnage, dans une étape de prélèvement de l'échantillon et des réactifs,

- la figure 7 illustre le fonctionnement de la vanne d'échantillonnage dans une étape de transfert et de mélange de l'échantillon avec les réactifs dans les cuves de mesure,
- la figure 8 présente une représentation schématique d'un dispositif selon l'invention, selon un second mode de réalisation.

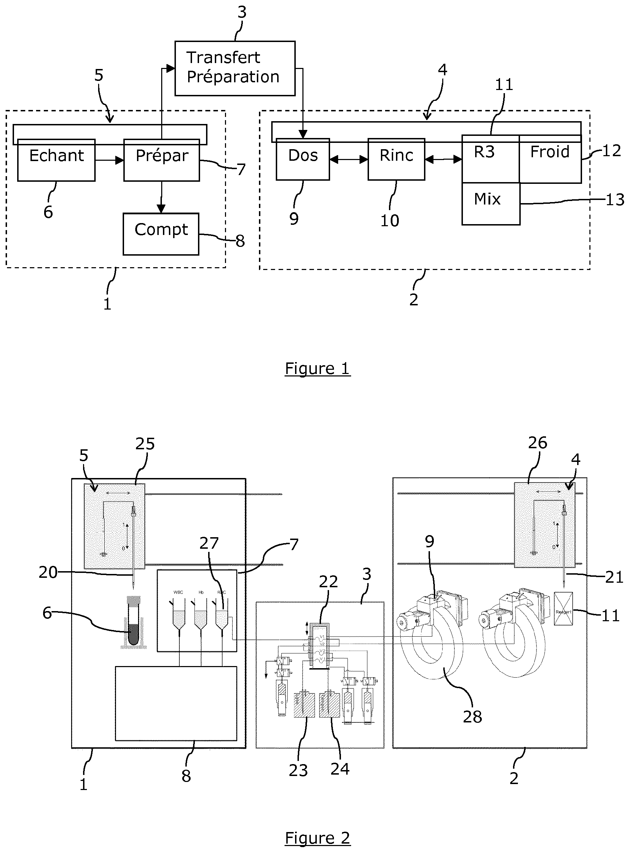

En référence aux figures 1 et 2, nous allons décrire un premier mode de réalisation d'un dispositif d'analyse permettant d'obtenir à partir d'un seul prélèvement de sang total 6 un hémogramme et le dosage d'au moins un analyte sanguin. Ce dispositif est conçu de telle sorte à permettre d'atteindre une cadence d'analyse élevée, de l'ordre d'au moins une analyse complète par minute.

Ce dispositif est composé d'un module d'hématologie 1 et d'un module d'immuno-détection 2. Ces deux modules sont couplés par l'intermédiaire d'une interface mécanique et fluidique, le module de transfert 3, qui leur permet d'utiliser un seul et même échantillon sanguin 6. Ils fonctionnent de manière parallèle. Cette représentation sous forme de modules est bien entendu purement fonctionnelle, pour faciliter la compréhension, et elle n'est en aucun cas limitative en ce qui concerne l'implémentation physique des composants.

Le module d'hématologie 1 est basé sur des principes et des technologies connus de l'homme du métier, telles que par exemple ceux décrits dans US 6 106 778. Ce module 1 est donc décrit ici de manière relativement succincte. Il est composé principalement de trois sous-ensembles qui sont les premiers moyens de prélèvement et de transfert 5, les premiers moyens de préparation 7 et les moyens de mesure d'hématologie 8.

Le dispositif peut comprendre un passeur d'échantillon qui permet d'amener, de manière automatique, un échantillon de sang contenu dans un tube 6.

Les premiers moyens de prélèvement et de transfert 5 comprennent une aiguille métallique creuse 20 connectée à un système de déplacement et d'aspiration, qui permet de prélever un échantillon de sang total dans le tube de prélèvement 6.

La surface de cette aiguille 20 est de préférence traitée par un traitement destiné à limiter l'adhérence des différents composés cellulaires et chimiques du sang.

Lorsque le dispositif est conçu pour fonctionner avec des tubes d'échantillon 6 hermétiquement fermés, cette aiguille 20 peut être équipée d'un système de perçage du bouchon. Dans ce cas, le dispositif comprend également un système d'équilibrage de la pression.

L'aiguille 20 est couplée à un chariot mobile 25 qui permet de la positionner au dessus des bacs des premiers moyens de préparation 7 et de distribuer les aliquotes de sang dans le module d'hématologie 1.

Les premiers moyens de préparation 7 comprennent plusieurs bacs de mélanges 27, connectés chacun à une ou plusieurs alimentations en réactifs spécifiques, une vidange, un circuit de bullage pour les mélanges et un circuit de transfert vers le dispositif de mesure d'hématologie 8. Ils permettent un mélange et un dosage précis de l'aliquote amenée par l'aiguille 20 avec différents réactifs, pour obtenir les mélanges nécessaires à l'établissement de l'hémogramme et à la numération.

Chaque bac 27 est associé à un ou plusieurs réactifs permettant un traitement spécifique de l'échantillon : dilution dans un réactif de tonicité égale à celle des globules rouges, lyse des globules rouges, lyse et formation d'un complexe stable de l'hémoglobine, .... Ces mélanges sont ensuite analysés par des dispositifs spécifiques qui comprennent des éléments fluidiques connus de l'homme du métier pour permettre la réalisation d'un hémogramme complet à la cadence recherchée. Ces bacs de dilution 27 et le dispositif de mesure d'hématologie 8 permettent ainsi le comptage et la différenciation des leucocytes, des érythrocytes et des thrombocytes. Ces données hématologiques comprennent d'autres paramètres tels que la mesure de l'hématocrite Hct ou les constantes de Wintrobe. Enfin, les leucocytes peuvent être différenciés en sous-populations cellulaires permettant le comptage des lymphocytes, monocytes, granuleux et/ou de cellules immatures.

Les moyens de mesure d'hématologie 8 sont basés sur des techniques connues de cytométrie en flux. Ils comprennent des moyens de déplacement pour faire passer les cellules sanguines, une à une, dans un ou plusieurs compteurs, et associent des mesures d'impédance à des mesures optiques. Le volume de mélange analysé est maîtrisé de façon à satisfaire les exigences de précision, en particulier pour les numérations. Le traitement de ces données permet l'obtention des résultats hématologiques.

Le module de transfert 3 assure l'interface entre le module d'hématologie 1 et le module d'immuno-détection 2. Il permet la récupération d'une ou plusieurs aliquote(s) de sang dans le module d'hématologie 1 et son (leur) transfert(s) au module d'immuno-détection 2.

Il permet également de poursuivre la préparation du mélange pour la mesure immunologique, par des dilutions supplémentaires et l'adjonction d'un ou plusieurs réactifs.

Le module de transfert 3 comprend des seconds moyens de transfert avec une vanne d'échantillonnage 22 qui permet de prélever un échantillon dans une cuve 27 au sein des premiers moyens de préparation 7. Il comprend en outre des seconds moyens de préparation 23, 24 pour préparer les échantillons pour le module d'immuno-détection 2.

Leur fonctionnement est détaillé plus loin.

Le module d'immuno-détection 2 comprend une cuve de mesure 9. Il comprend également des seconds moyens de préparation 10, 11 avec un ensemble de réactifs équipés de leurs moyens de distribution et un compartiment régulé en température 11 permettant le stockage du réactif de dosage R3 contenant les particules magnétiques. Il comprend en outre des seconds moyens de transfert 4 avec une aiguille spécifique 21 montée sur un chariot 26 ou sur un bras motorisé (non représenté). Il comporte également une cuve de rinçage 10 de l'aiguille 21.

La cuve de mesure 9 est équipée d'une optique spécifique permettant de faire une mesure de densité optique dans le milieu, éventuellement compensée par une mesure de la puissance de la source lumineuse. Elle est munie d'un électroaimant 28 réalisé avec une bobine, qui permet l'application d'un champ magnétique au milieu mesuré. L'application de ce champ et son amplitude en fonction du temps définissent le cycle de magnétisation. Il est piloté par un dispositif électronique permettant l'annulation du champ coercitif si nécessaire, et il est synchronisé avec la mesure de variation de densité optique. Le profil de ce cycle dépend du type de mesure immunologique effectuée. Le principe de mesure est détaillé plus précisément plus loin.

La cuve de mesure 9 est maintenue dans une ambiance régulée en température. Elle est équipée d'un système de chauffage d'appoint par exemple avec un bloc d'aluminium chauffé placé autour de cette cuve 9 dans les zones disponibles. Une sonde de température permet de mesurer sa température qui pourra ensuite être régulée si nécessaire.

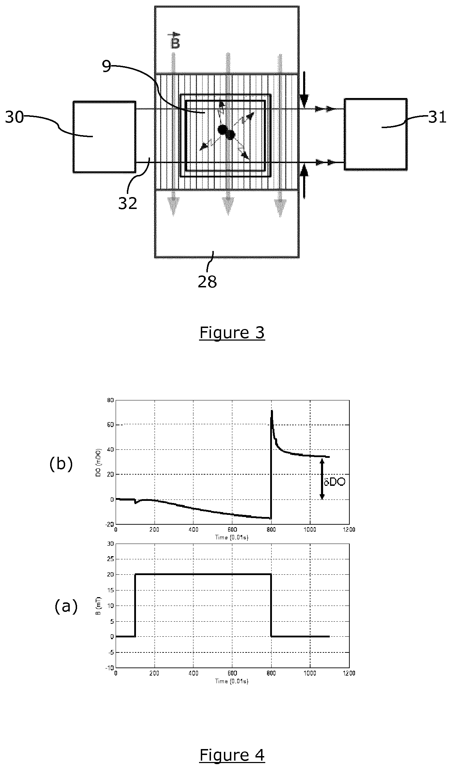

En référence à la figure 3, cette cuve de mesure 9 a une contenance de l'ordre de quelques centaines de µL. Elle comporte deux surfaces planes transparentes opposées dans le sens de passage du faisceau lumineux 32. Son volume est conçu pour maximiser le volume éclairé. De façon préférentielle, elle peut comporter un entonnoir de vidange permettant un rinçage et un séchage efficaces. En fonction des applications, elle peut être réalisée dans différents matériaux tels que du verre, du quartz ou du plastique injecté ou usiné comme par exemple du PMMA ou du polyamide... Elle subit de préférence un traitement physicochimique de surface afin de réduire l'adhérence des différents composés cellulaires et chimiques du sang.

Le compartiment régulé en température 11 permet de maintenir le réactif R3 utilisant les particules magnétiques à une température de conservation de quelques degrés (typiquement entre 5 et 15 °C), ce qui permet de le stocker au sein même de l'analyseur. Il est équipé d'un système d'agitation par exemple un système d'agitation ultrasonore 13, qui permet la mise et le maintien en suspension des particules colloïdales dans leur solution, ainsi que la dissociation d'éventuels agrégats dûs à des interactions non spécifiques. Ce système d'agitation ultrasonore 13 comprend une sonotrode (i.e. une pièce qui transmet les ultrasons) externe au flacon de réactif, et couplée mécaniquement à ce dernier. D'autres systèmes d'agitation connus de l'homme du métier peuvent bien entendu être envisagés.

Au sein de cette zone régulée en température 11, peuvent être aussi stockés les réactifs de contrôle ou de calibration du module de mesure immunologique 2.

Un système de régulation de la température 12 permet le maintien en température du compartiment 11. Il n'est pas nécessaire d'avoir une grande précision. Les systèmes classiquement utilisés dans l'industrie et connus de l'homme de l'art sont largement suffisants. Par exemple ce peut être un module à effet Peltier piloté par une boucle de régulation tout-ou-rien.

L'aiguille de prélèvement spécifique 21 est dédiée au module d'immuno-détection 2. Cela est nécessaire pour pouvoir garantir la cadence des cycles, le taux d'occupation de l'aiguille 20 du module d'hématologie 1 étant élevé dans un analyseur à forte cadence. Dans la configuration présentée ici, elle est associée à des actionneurs et à un chariot 26 permettant son déplacement vertical et horizontal au-dessus de la cuve de mesure 9, du flacon de particules magnétiques 11 et de la cuve de rinçage 10.

Cette aiguille de prélèvement 21 est utilisée pour le prélèvement des particules magnétiques et leur distribution dans la solution analysée, ce qui limite les risques de contamination du réactif de dosage R3. Elle peut être aussi utilisée pour effectuer le mélange par aspiration et refoulements successifs du mélange dans la cuve de mesure 9.

Les réactifs mis en oeuvre dans le module d'immuno-détection 2 assurent trois fonctions principales que sont :

- la lyse des cellules sanguines,
- le maintien d'un pH à une valeur stable et définie
- la réaction d'agglutination.

Nous appellerons R1 le réactif lytique, R2 la solution tampon et R3 le réactif de dosage contenant les particules magnétiques.

Le réactif R1 comprend un agent lytique qui comprend un détergent ou toute autre molécule ayant des propriétés d'altération et de fragmentation des membranes cellulaires. Il peut comprendre par exemple un détergent de la classe des saponines, ou bien de la classe des ammoniums quaternaires. Il peut également comprendre un détergent anionique choisi parmi la classe des acides biliaires.

La solution tampon R2 comprend un système tampon capable de maintenir un pH constant pendant le déroulement de la réaction. Ce peut être par exemple un tampon glycine 50mM, avec un pH de l'ordre de 8,5.

Le réactif lytique R1 et la solution tampon R2 peuvent être combinés en un seul réactif.

Le réactif de dosage R3 comprend des particules submicrométriques à la surface desquelles sont greffés des anticorps dirigés contre l'analyte à doser. Ces particules sont caractérisées par un diamètre moyen D et un écart type $\sigma_D$ telles que $\sigma_D/D < 20$ %, le diamètre moyen étant compris en 100 et 300 nm. Ces particules, de forme sensiblement sphérique, sont formées d'une coque en matériau polymère dont l'épaisseur est de quelques dizaines de nanomètres au sein de laquelle se trouve un noyau à forte teneur en oxyde de fer. Des exemples d'un tel réactif R3 peuvent être trouvés dans EP 1 446 666.

La détection des agrégats particulaires est réalisée par un dispositif optique optimisé pour détecter la lumière diffusée par le mélange colloïdal. Il comprend une source de lumière 30 de type diode électroluminescente (DEL) et un détecteur 31 avec une photodiode ou un photomultiplicateur placés de part et d'autre de la cuve de mesure 9. Il comprend en outre des moyens de conditionnement de faisceau qui permettent de générer au travers de la cuve 9 un faisceau de lumière 32 collimaté, dont la divergence est minimale et idéalement limitée par la diffraction.

De manière générale, il est préférable d'utiliser une source de lumière 30 de faible cohérence temporelle, pour réduire ou éviter divers bruits optiques comme le phénomène de granularité laser ou encore les interférences multiples dans le milieu d'analyse, résultant de réflexions multiples sur les parois du montage.

En référence à la figure 4, pour effectuer une mesure de dosage on effectue un cycle de magnétisation, qui comprend l'application d'un champ magnétique avec un profil temporel défini dans sa forme, sa durée, son nombre de périodes,.... Dans l'exemple présenté, ce cycle de magnétisation comprend l'application d'un échelon temporel de champ magnétique, tel qu'illustré à la figure 4(a). Durant l'application du champ magnétique, les particules magnétiques colloïdales se regroupent en chaînes. Après l'interruption du champ magnétique, elles se dispersent de nouveau à l'exception des agrégats constitués par la captation de l'analyte ou de la protéine d'intérêt par les ligands ou les anticorps.

On effectue un enregistrement du signal optique transmis au travers de la cuve de mesure 9 avant, pendant et après le cycle de magnétisation, tel qu'illustré à la figure 4(b). On détermine ensuite une variation de la lumière transmise avant et après l'application du champ magnétique en calculant une variation de densité optique $\delta OD = -\text{Log}(It/Io)$ où « Log » est l'opération de logarithme, It est l'intensité de la lumière transmise après application du champ magnétique et Io est l'intensité de la lumière transmise avant application du champ magnétique. Cette variation de lumière transmise est due à la diffusion des agrégats qui se sont constitués.

Pour chaque analyte dosé noté i de concentration Ci, le procédé permet de déterminer une variation $\delta ODi$. En pratique, la quantité mesurée $\delta ODi$ n'est pas directement proportionnelle à Ci. Une fonction mathématique, qui peut être un polynôme dont les coefficients sont déterminés par étalonnage, permet de déterminer Ci par une relation de la forme $Ci = Fi(\delta ODi)$, où Fi est une fonction spécifique à l'analyte Ci.

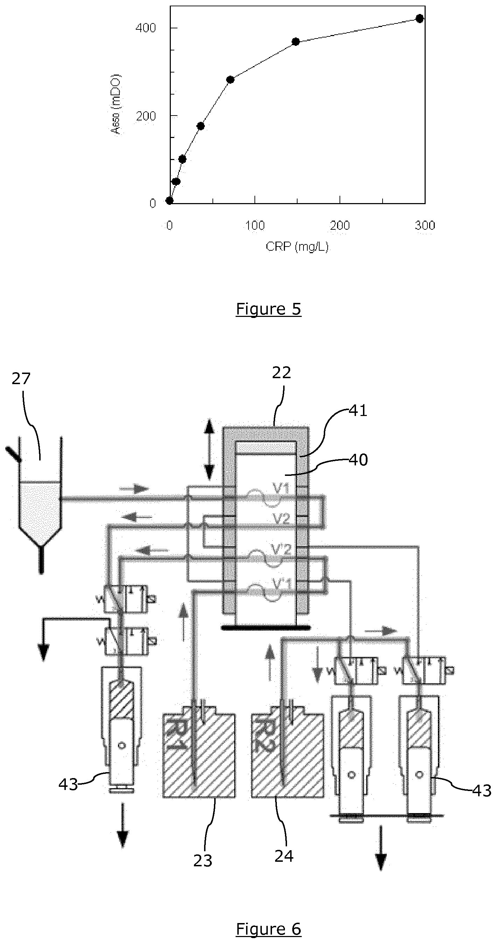

La figure 5 présente un exemple de fonction d'étalonnage pour le dosage de la protéine C-réactive dans un échantillon de sang total.

On va décrire maintenant plus précisément un exemple de mise en oeuvre du procédé selon l'invention pour le dosage de deux analytes différents.

Le module d'hématologie 1 est conçu de telle sorte à pouvoir effectuer 80 tests à l'heure.

La durée d'un cycle pour obtenir l'hémogramme complet ainsi que la numération des éléments du sang est de 45 secondes.

Le module d'immuno-détection 2 comprend deux cuves de mesure 9, qui permettent de faire deux mesures simultanées, en 45 secondes également. Ces mesures peuvent comprendre des mesures sur deux analytes différents, ou des mesures sur un même analyte sur deux plages de mesures différentes.

Le module de transfert 3 permet de prélever une dilution initialement préparée par le module d'hématologie 1 pour le comptage des globules rouges, à travers une vanne d'échantillonnage 22. Il alimente ensuite simultanément les deux cuves de mesure 9, avec deux préparations différentes d'un même échantillon de sang.

Le dispositif exécute la séquence temporelle suivante :
Le module d'hématologie 1 prélève l'aliquote de sang 6 et rince son aiguille 20 de 0 à 10s. En parallèle, les différentes cuves des 2 modules sont rincées. Ensuite le module d'hématologie 1 prépare la première dilution pour la numération des globules rouges (RBC), pendant que le module d'immuno-détection 2 prélève les réactifs de dosage R3 et commence à les distribuer dans ses deux cuves de mesure 9 (chaque cuve recevant un réactif de dosage R3 différent, spécifique d'une protéine). Après une vingtaine de secondes, une partie de la dilution RBC est transférée vers le module d'immuno-détection 2, tandis que celui-ci termine la distribution des réactifs de dosage R3. Le module d'hématologie 1 poursuit en parallèle la préparation des autres mélanges dans ses autres bacs 27. On est alors à peu près à 25s du début du cycle et le module d'hématologie termine son cycle de son côté jusqu'à 45s. Pendant ce temps, les solutions dans les cuves de mesure 9 du module d'immuno-détection 2 sont mélangées puis la mesure de dosage commence, pour se terminer à 45s du début du cycle.

Il est à noter que le cycle de mesure de dosage ne dure que 9 secondes, et la cadence est de fait limitée par la durés du cycle de préparation. Le procédé selon l'invention est donc compatible avec des cadences de mesures nettement plus élevées, en mettant en oeuvre des systèmes de préparation adaptés, par exemple à base de systèmes microfluidiques.

La vanne d'échantillonnage 22 mise en oeuvre dans ce mode de réalisation est une vanne d'échantillonnage de type tiroir, bien connue de l'homme du métier. Elle comprend une pièce mobile 40 en translation dans un support 41. La pièce mobile 40 est traversée de capillaires qui permettent de stocker des aliquotes, et le support 41 comprend des ports fluidiques. Une aliquote peut être aspirée par un premier port fluidique et stockée dans la pièce mobile 40, puis la pièce mobile 40 est déplacée de telle sorte que l'aliquote puisse être expulsée par un autre port fluidique.

La vanne d'échantillonnage 22 gère deux types d'aliquotes :

- une première série d'aliquotes correspondants au prélèvement de plusieurs volumes déterminés d'une dilution d'échantillon contenue au sein du module d'hématologie : ces volumes prélevés sont désignés v1, v2.
- une seconde série d'aliquotes correspondants au prélèvement de plusieurs quantités d'un réactif lytique R1 : ces volumes prélevés sont désignés v'1, v'2.

Chaque volume v'i est adapté pour effectuer la lyse totale des cellules contenues dans chaque aliquote vi. La vanne d'échantillonnage 22 est conçue pour coupler fluidiquement les aliquotes vi et v'i.

En référence à la figure 6, dans un premier temps, la vanne d'échantillonnage 22 est positionnée de telle sorte à permettre le prélèvement d'un échantillon dans le bac 27, et du réactif lytique R1 dans un bac 23. Ces prélèvements sont effectués par pompage par des seringues 43 qui prélèvent également de la solution tampon R2 dans un bac 24.

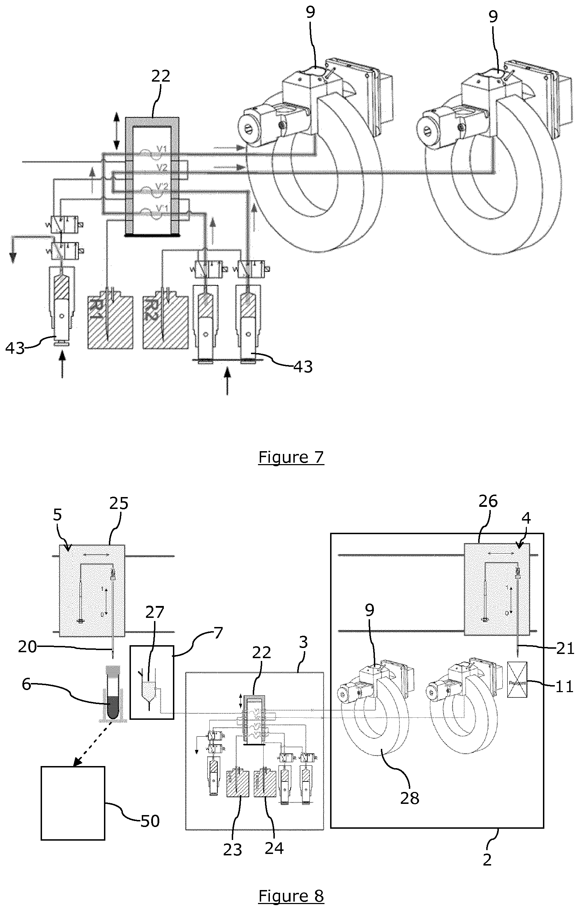

En référence à la figure 7, dans un second temps la vanne d'échantillonnage 22 est positionnée de telle sorte à permettre que les aliquotes vi et v'i soient poussées dans les cuves de mesure 9 par la solution tampon R2 dont un volume v"i, adapté pour obtenir le taux de dilution final recherché est également introduit dans les cuves de mesure 9. Cette poussée est effectuée par les seringues 43.

En outre, un volume v"'i de réactif de dosage R3 (spécifique à la protéine recherchée) est distribué dans les cuves de mesure 9 par l'aiguille de prélèvement 21 du module d'immuno-détection 2.

Pour le dosage d'un analyte tel que la protéine C réactive ou CRP :

- le réactif lytique R1 est une solution aqueuse d'un détergent capable de lyser rapidement les éléments cellulaires de l'échantillon. Le détergent peut être ionique ou non ionique avec une préférence pour la saponine ;
- le tampon R2 est un système tampon capable de maintenir un pH de de 8,5 dans le milieu réactionnel comme par exemple un tampon glycine ;
- les réactifs de dosage R3 comprennent une suspension de particules sur lesquelles est greffé de façon covalente un anticorps monoclonal ou polyclonal capable de reconnaître spécifiquement l'analyte à doser (ici la CRP). Eventuellement, une suspension peut contenir plusieurs populations de particules ayant chacune immobilisé à leur surface un anticorps différent (monoclonal ou polyclonal), chacun reconnaissant un épitope différent de l'analyte à doser.

Un exemple de protocole pour une gamme de 2 à 200mg/L de CRP est le suivant :

v1 = 6,5 $\mu$l d'échantillon prédilué dans le bac de mesure des globules blancs du module d'hématologie (1/40)
v'1 = 100 $\mu$l de saponine à 0,2% dans l'eau distillée
v"1 = 118 $\mu$l de tampon glycine 0,1M pH 8,5
v"'1 = 25 $\mu$l d'une suspension de particules à 0,4%

Suivant des variantes de modes de réalisation :

- Le module de prélèvement et de transfert 5 peut comprendre une vanne d'échantillonnage ou un système d'échantillonnage comme par exemple décrit dans le document WO 2009/024710 connectés à un système de distribution. Il peut également comprendre un tube capillaire au lieu d'une aiguille 20 ;
- il est possible de prélever avec les seconds moyens de transfert (ou le module de transfert) directement dans un ou plusieurs bacs 27, une ou plusieurs dilutions initialement préparées pour une mesure hématologique. Ce prélèvement peut être effectué dans n'importe quel bac, en fonction du taux de dilution et des réactifs utilisés. Ce peut être aussi dans un bac dédié à la mesure immunologique. La configuration est choisie en fonction de deux critères principaux. Tout d'abord le mélange transféré doit satisfaire les besoins de la mesure immunologique (comme par exemple la compatibilité des réactifs entre eux, des taux de dilutions...). Ensuite le procédé doit être peu invasif vis-à-vis du module hématologique 1, en particulier il ne doit pas rallonger sensiblement la durée de son cycle. Bien évidemment il ne doit pas non plus dégrader les dilutions nécessaires au module d'hématologie 1.

- la cuve de mesure 9 peut comprendre un système de mélange de la solution analysée. Il peut être assuré indifféremment par un circuit de bullage, par un circuit d'aspirations et de refoulements successifs dans un tuyau ou une chambre dédiée ;

- la cuve de mesure 9 peut ne pas être régulée en température. Elle peut comprendre un ou plusieurs capteurs de température utilisés pour corriger les mesures d'immuno-détection, en appliquant des modèles ou des algorithmes adaptés ;

- Le système d'agitation (ultrasonore) 13 peut être réalisé de différentes façons. Ce peut être de manière non invasive avec une agitation extérieure au flacon, par l'intermédiaire d'un couplage sec ou humide entre le flacon et une sonotrode. Il est possible aussi d'utiliser une sonotrode immergée, sous forme d'aiguille vibrante, ou même d'utiliser l'aiguille de prélèvement pour réaliser l'agitation ;

- Les seconds moyens de transfert 4 peuvent être conçus de telle sorte à permettre d'amener l'aiguille de prélèvement 21 dans un bac 27 du module d'hématologie 1. Dans ce cas le support du chariot horizontal 26 peut être fixé sur le même support que le chariot 25 du module d'hématologie 1. Il peut être aussi fixé sur des supports séparés, alignés ou non sur les axes d'alignement des cuves 27 du module d'hématologie, du moment qu'il est aligné avec le bac d'intérêt. Le chariot horizontal peut aussi être remplacé par un bras rotatif. Dans ce cas la cuve de mesure 9, la cuve de rinçage 10, le flacon de particules 11 et l'éventuelle cuve du module hématologique 27 doivent être alignés de façon sensiblement circulaire autour du centre de rotation de ce bras ;

- Le réactif lytique R1 peut être omis dans les cas où l'échantillon est d'abord prédilué dans un des bacs du module d'hématologie, en particulier le bac servant au comptage des globules blancs. Dans ce cas, l'échantillon de sang total peut être mélangé avec le réactif lytique du module d'hématologie avant d'être transféré dans le module d'immuno-détection. Dans ce cas également, l'échantillon ne reçoit que la solution tampon R2 et le réactif de dosage R3 dans le module d'immuno-détection ;

- La source de lumière 30 peut comprendre toutes sources de faible dimension telle qu'un laser, une diode super-luminescente, une RCLED (diode électroluminescente à cavité résonnante) ou une lampe à incandescence judicieusement diaphragmée. Il est également possible de générer le faisceau 32 par collimation d'un faisceau de lumière issu d'une fibre optique monomode.

En référence à la figure 8, nous allons maintenant décrire un dispositif d'analyse, qui ne fait pas partie de l'invention, permettant d'obtenir à partir d'un seul prélèvement de sang total 6 un hémogramme et le dosage d'au moins un analyte sanguin. Ce dispositif est conçu de telle sorte à permettre d'atteindre une cadence d'analyse élevée, de l'ordre d'au moins une analyse complète par minute.

Ce dispositif comprend un module d'immuno-détection 2 et un module d'hématologie externe 50. Le module d'hématologie externe 50 fonctionne de manière similaire au module d'hématologie 1 du premier mode de réalisation, et le module d'immuno-détection 2 est identique. Aussi, seules les différences entre les premiers et seconds modes de réalisation seront détaillées dans ce qui suit.

Dans ce mode de réalisation, c'est le prélèvement de sang total 6 qui est transféré entre le module d'immuno-détection 2 et le module d'hématologie externe 50, et non pas, comme dans le premier mode de réalisation, une prédilution. Les mesures d'hémogramme et de dosage d'analyte(s) sanguin(s) sont donc toujours effectuées sur le même prélèvement de sang total 6.

Le dispositif peut comprendre un passeur d'échantillon qui permet d'amener de transférer de manière automatique l'échantillon de sang contenu dans un tube 6 entre le module d'immuno-détection 2 et le module d'hématologie externe 50.

Le couplage des mesures d'hématologie et d'immuno-détection ne se limite pas à un partage du prélèvement de sang total 6. Les informations sont également transmises entre les modules de telle sorte à pouvoir utiliser les mesures d'hématologie pour traiter les mesures d'immuno-détection, tel que décrit précédemment. Pour cela, le module d'immuno-détection 2 et le module d'hématologie externe 50 peuvent être interconnectés par un réseau informatique.

Le dispositif selon l'invention comprend toujours des premiers moyens de prélèvement et de transfert 5 et des premiers moyens de préparation 7 tels que décrits précédemment, mais qui sont fonctionnellement rattachés au module d'immuno-détection 2. De la même manière, le module de transfert 3, identique à celui du premier mode de réalisation, est fonctionnellement rattachés au module d'immuno-détection 2.

Les premiers moyens de préparation 7 comprennent une cuve 27 qui permet d'effectuer une première dilution de l'aliquote amenée par l'aiguille 20 à partir du prélèvement de sang total 6.

Le module de transfert 3 comprend des seconds moyens de transfert avec une vanne d'échantillonnage 22 qui permet de prélever un échantillon dans la cuve 27 au sein des premiers moyens de préparation 7.

Ainsi, ce mode de réalisation permet, comme précédemment, d'effectuer des mesures d'immuno-détection avec des taux de dilution élevés tout en minimisant les risques de contamination croisée entre l'échantillon de sang 6 grâce à l'utilisation de la cuve intermédiaire 27.

Il est à noter enfin que le module d'hématologie externe 50 comprend ses propres moyens de prélèvement et de préparation qui sont distincts des premiers moyens de prélèvement et de transfert 5 et des premiers moyens de préparation 7 rattachés au module d'immuno-détection 2.

Suivant des variantes,

- il est possible de coupler un module d'immuno-détection 2 avec plusieurs modules d'hématologie externes 50 ;
- il est possible de coupler un module d'immuno-détection 2 avec un ou plusieurs module(s) d'hématologie externe(s) 50 et un module d'hématologie 1 tel que présenté dans le premier mode de réalisation.

Bien sûr, l'invention n'est pas limitée aux exemples qui viennent d'être décrits et de nombreux aménagements peuvent être apportés à ces exemples sans sortir du cadre de l'invention.

**Revendications**

1. Dispositif d'analyse de paramètres biologiques à partir d'un échantillon biologique (6), comprenant :

   - des premiers moyens de transfert (5, 20, 25) aptes à transférer au moins en partie ledit échantillon biologique (6) vers des premiers moyens de préparation (7),
   - des premiers moyens de préparation (7) aptes à réaliser au moins une dilution dudit échantillon biologique (6) avec au moins un diluant et/ou un réactif,
   - des seconds moyens de préparation (10, 11, 22, 23, 24) aptes à effectuer sur un premier échantillon issu des premiers moyens de préparation (7) au moins une dilution avec un réactif de dosage (R3) comprenant des particules fonctionnalisées en surface avec au moins un ligand spécifique d'au moins un analyte d'intérêt,
   - des moyens de mesure d'immuno-détection (30, 31) aptes à effectuer sur un échantillon issu des seconds moyens de préparation (10, 11, 22, 23, 24) un dosage d'au moins un analyte d'intérêt par mesure du taux d'agrégation de particules fonctionnalisées dans une cuve de mesure (9), **caractérisé en ce qu'**il comprend en outre :
   - des seconds moyens de transfert (4, 21, 22, 26) au moins en partie distincts des premiers moyens de transfert (5, 20, 25) et aptes à prélever ledit premier échantillon préalablement dilué dans les premiers moyens de préparation (7), et à le transférer vers les seconds moyens de préparation (10, 11, 22, 23, 24),
   - des moyens d'application d'un champ magnétique (28) dans ladite cuve de mesure (9) aptes à provoquer par interaction magnétique une accélération de l'agrégation desdites particules fonctionnalisées, lesquelles comprenant des particules colloïdales magnétiques, et

   des moyens de mesure de composante cellulaire (8) aptes à fournir à partir d'un second échantillon issu des premiers moyens de préparation (7) au moins une mesure de volume de cellules par rapport au volume total.

2. Le dispositif de la revendication 1, qui comprend en outre des moyens de mesure de composante cellulaire (8, 50) aptes à fournir à partir de l'échantillon biologique (6) au moins une mesure de volume de cellules par rapport au volume total.

3. Le dispositif de l'une des revendications précédentes, dans lequel les particules fonctionnalisées comprennent des particules pourvues d'un noyau à forte teneur en oxyde de fer entouré d'une coque en matériau polymère.

4. Le dispositif de l'une des revendications précédentes, dans lequel les particules fonctionnalisées comprennent des particules de forme essentiellement sphérique d'un diamètre moyen inférieur à 1 micromètre.

5. Le dispositif de l'une des revendications précédentes, dans lequel les moyens de mesure d'immuno-détection (2) comprennent des moyens de mesure optique avec au moins une source de lumière (30) et au moins un détecteur (31) placés à proximité de la cuve de mesure (9), laquelle étant pourvue, au moins au niveau desdits moyens de mesure optique (30, 31), de parois sensiblement transparentes.

6. Le dispositif de la revendication 5, dans lequel les moyens de mesure d'immuno-détection comprennent en outre des moyens de conditionnement optique aptes à produire à partir d'au moins une source de lumière (30) un faisceau de lumière collimaté (32) traversant la cuve de mesure (9).

7. Le dispositif de l'une des revendications 5 ou 6, qui comprend au moins une source de lumière (30) apte à émettre dans des longueurs d'onde optiques comprises entre 600 nanomètres et 900 nanomètres.

8. Le dispositif de l'une des revendications précédentes, qui comprend en outre un électroaimant (28) apte à produire un champ magnétique dans la cuve de mesure (9).

9. Le dispositif de l'une des revendications précédentes, qui comprend en outre des moyens de régulation de la température de la cuve de mesure (9).

10. Le dispositif de l'une des revendications précédentes, qui comprend en outre un conteneur de stockage (11) régulé à une température optimale pour stocker le réactif de dosage (R3).

11. Le dispositif de la revendication 10, qui comprend en outre des moyens d'agitation par ultrasons (13) pour mettre et/ou maintenir en suspension les particules fonctionnalisées dans le réactif de dosage (R3) stocké, comprenant au moins l'un des moyens suivants : une sonotrode externe couplée au conteneur de stockage, une sonotrode immergée dans le réactif de dosage (R3).

12. Le dispositif de l'une des revendications précédentes, dans lequel les premiers moyens de transfert comprennent une aiguille de prélèvement (20) et des moyens de déplacement (25) de ladite aiguille de prélèvement (20).

13. Le dispositif de l'une des revendications précédentes, dans lequel les seconds moyens de transfert comprennent une aiguille de prélèvement (21) et des moyens de déplacement (26) de ladite aiguille de prélèvement (21) aptes à transférer du réactif de dosage (R3) dans la cuve de mesure (9).

14. Le dispositif de l'une des revendications précédentes, dans lequel les seconds moyens de transfert comprennent une vanne d'échantillonnage (22) apte à prélever un échantillon dans les premiers moyens de préparation (7).

15. Le dispositif de l'une des revendications précédentes, pour d'analyse de paramètres biologiques à partir d'un échantillon biologique comprenant un échantillon de sang total (6).

16. Le dispositif de la revendication 15, qui comprend des moyens de mesure de composante cellulaire (8, 50) aptes à fournir au moins une mesure d'hématocrite.

17. Procédé d'analyse de paramètres biologiques à partir d'un échantillon biologique (6), comprenant des étapes de :

- transfert par des premiers moyens de transfert (5, 20, 25) d'au moins une partie dudit échantillon biologique (6) vers des premiers moyens de préparation (7),
- réalisation par des premiers moyens de préparation (7) d'au moins une dilution dudit échantillon biologique (6) avec au moins un diluant et/ou un réactif,
- réalisation par des seconds moyens de préparation (10, 11, 22, 23, 24), sur un premier échantillon issu des premiers moyens de préparation (7), d'au moins une dilution avec un réactif de dosage (R3) comprenant des particules fonctionnalisées en surface avec au moins un ligand spécifique d'au moins un analyte d'intérêt,
- dosage par des moyens de mesure d'immuno-détection (30, 31) d'au moins un analyte d'intérêt avec un échantillon issu des seconds moyens de préparation (10, 11, 22, 23, 24), par mesure dans une cuve de mesure (9) du taux d'agrégation de particules fonctionnalisées,

**caractérisé en ce qu'**il comprend en outre des étapes de :

- prélèvement par des seconds moyens de transfert (4, 21, 22, 26) au moins en partie distincts des premiers moyens de transfert (5, 20, 25) dudit premier échantillon préalablement dilué dans les premiers moyens de préparation (7), et transfert vers les seconds moyens de préparation (10, 11, 22, 23, 24),
- application d'un champ magnétique dans ladite cuve de mesure (9) de telle sorte à provoquer par interaction magnétique une accélération de l'agrégation desdites particules fonctionnalisées, lesquelles comprenant des particules colloïdales magnétiques, et

une étape d'obtention par des moyens de mesure de composante cellulaire (8), à partir d'un second échantillon issu des premiers moyens de préparation (7), d'au moins une mesure de volume de cellules par rapport au volume total.

18. Le procédé de la revendication 17, qui comprend en outre une étape d'obtention par des moyens de mesure de composante cellulaire (8, 50), à partir de l'échantillon biologique (6), d'au moins une mesure de volume de cellules par rapport au volume total.

**19.** Le procédé de l'une des revendications 17 ou 18, dans lequel le dosage d'au moins un analyte d'intérêt comprend des étapes de :

- introduction dans la cuve de mesure (9) d'une solution de mesure comprenant au moins un échantillon et du réactif de dosage (R3),
- mesure d'une première intensité optique au travers de la cuve de mesure,
- application d'un champ magnétique dans la cuve de mesure (9) pendant une durée déterminée, de telle sorte à permettre l'agrégation des particules fonctionnalisées sous l'effet du champ magnétique,
- après l'arrêt du champ magnétique, mesure d'une seconde intensité optique représentative de l'agrégation résiduelle des particules fonctionnalisées due aux couplages entre ligand et analyte d'intérêt,
- calcul d'une variation de densité optique en fonction du ratio desdites première et seconde intensités optiques, et
- application d'une fonction d'étalonnage préalablement déterminée pour calculer la concentration de la protéine d'intérêt à partir de la variation de densité optique.

**20.** Le procédé de la revendication 19, dans lequel l'échantillon biologique est dilué à un taux supérieur à x500 dans la solution de mesure.

**21.** Le procédé de l'une des revendications 17 à 20, pour d'analyse de paramètres biologiques à partir d'un échantillon biologique (6) qui comprend un échantillon de sang total.

**22.** Le procédé de la revendication 21, qui met en oeuvre pour le dosage de l'analyte d'intérêt :

- un réactif lytique (R1) comprenant de la saponine,
- une solution tampon (R2) apte à maintenir un pH optimal.

**23.** Le procédé de l'une des revendications 21 ou 22, qui met en oeuvre des particules fonctionnalisées avec un ligand apte à permettre un dosage d'un analyte d'intérêt qui est la protéine C-réactive (CRP).

**Patentansprüche**

**1.** Vorrichtung zur Analyse biologischer Parameter ausgehend von einer biologischen Probe (6), umfassend:

- erste Überführungsmittel (5, 20, 25), die dafür ausgelegt sind, die biologische Probe (6) zumindest zum Teil zu ersten Zubereitungsmitteln (7) zu überführen,
- erste Zubereitungsmittel (7), die dafür ausgelegt sind, mindestens eine Verdünnung der biologischen Probe (6) mit mindestens einem Verdünnungsmittel und/oder einem Reagenz durchzuführen,
- zweite Zubereitungsmittel (10, 11, 22, 23, 24), die dafür ausgelegt sind, an einer ersten aus den ersten Zubereitungsmitteln (7) stammenden Probe mindestens eine Verdünnung mit einem Nachweisreagenz (R3) durchzuführen, das Partikel umfasst, die an der Oberfläche mit mindestens einem für mindestens einen Analyten von Interesse spezifischen Liganden funktionalisiert sind,
- Immunnachweis-Messmittel (30, 31), die dafür ausgelegt sind, an einer aus den zweiten Zubereitungsmitteln (10, 11, 22, 23, 24) stammenden Probe einen Nachweis mindestens eines Analyten von Interesse durch Messen des Anteils an Aggregation funktionalisierter Partikel in einer Messküvette (9) durchzuführen,

**dadurch gekennzeichnet, dass** es außerdem Folgendes umfasst:

- zweite Überführungsmittel (4, 21, 22, 26), die zumindest zum Teil von den ersten Überführungsmitteln (5, 20, 25) verschieden und dafür ausgelegt sind, die zuvor in den ersten Zubereitungsmitteln (7) verdünnte erste Probe aufzunehmen und sie zu den zweiten Zubereitungsmitteln (10, 11, 22, 23, 24) zu überführen,
- Mittel zum Anlegen eines Magnetfelds (28) in der Messküvette (9), die dafür ausgelegt sind, durch magnetische Wechselwirkung eine Beschleunigung der Aggregation der funktionalisierten Partikel, die kolloidale magnetische Partikel umfassen, hervorzurufen, und

Mittel zum Messen eines Zellbestandteils (8), die dafür ausgelegt sind, ausgehend von einer zweiten aus den ersten Zubereitungsmitteln (7) stammenden Probe mindestens eine Messung des Zellvolumens in Bezug auf das Gesamtvolumen bereitzustellen.

2. Vorrichtung nach Anspruch 1, die außerdem Mittel zum Messen einer Zellkomponente (8, 50) umfasst, die dafür ausgelegt sind, ausgehend von der biologischen Probe (6) mindestens eine Messung des Zellvolumens in Bezug auf das Gesamtvolumen bereitzustellen.

3. Vorrichtung nach einem der vorhergehenden Ansprüche, wobei die funktionalisierten Partikel Partikel umfassen, die mit einem Kern mit hohem Eisenoxidgehalt, umgeben von einer Hülle aus Polymermaterial, versehen sind.

4. Vorrichtung nach einem der vorhergehenden Ansprüche, wobei die funktionalisierten Partikel Partikel mit einer im Wesentlichen sphärischen Form mit einem mittleren Durchmesser von weniger als 1 Mikrometer umfassen.

5. Vorrichtung nach einem der vorhergehenden Ansprüche, wobei die Immunnachweis-Messmittel (2) Mittel zur optischen Messung mit mindestens einer Lichtquelle (30) und mindestens einem Detektor (31) umfassen, die in der Nähe der Messküvette (9) angeordnet sind, die zumindest in Höhe der optischen Messmittel (30, 31) im Wesentlichen transparente Wände besitzt.

6. Vorrichtung nach Anspruch 5, wobei die Immunnachweis-Messmittel außerdem Mittel zur optischen Konditionierung umfassen, die dafür ausgelegt sind, ausgehend von mindestens einer Lichtquelle (30) einen kollimierten Lichtstrahl (32) zu erzeugen, der durch die Messküvette (9) hindurchgeht.

7. Vorrichtung nach einem der Ansprüche 5 oder 6, die mindestens eine Lichtquelle (30) umfasst, die dafür ausgelegt ist, optische Wellenlängen zwischen 600 Nanometern und 900 Nanometern zu emittieren.

8. Vorrichtung nach einem der vorhergehenden Ansprüche, die außerdem einen Elektromagneten (28) umfasst, der zum Erzeugen eines Magnetfelds in der Messküvette (9) ausgelegt ist.

9. Vorrichtung nach einem der vorhergehenden Ansprüche, die außerdem Mittel zum Regulieren der Temperatur der Messküvette (9) umfasst.

10. Vorrichtung nach einem der vorhergehenden Ansprüche, die außerdem einen Vorratsbehälter (11) umfasst, der auf eine optimale Temperatur zum Aufbewahren des Nachweisreagenzes (R3) eingestellt ist.

11. Vorrichtung nach Anspruch 10, die außerdem Ultraschallbewegungsmittel (13) umfasst, mit denen die funktionalisierten Partikel im aufbewahrten Nachweisreagenz (R3) in Suspension gebracht und/oder gehalten werden, umfassend mindestens eines der folgenden Mittel: eine mit dem Vorratsbehälter verbundene externe Sonotrode, eine in das Nachweisreagenz (R3) eingetauchte Sonotrode.

12. Vorrichtung nach einem der vorhergehenden Ansprüche, wobei die ersten Überführungsmittel eine Probennahmenadel (20) und Mittel (25) zum Versetzen der Probennahmenadel (20) umfassen.

13. Vorrichtung nach einem der vorhergehenden Ansprüche, wobei die zweiten Überführungsmittel eine Probennahmenadel (21) sowie Mittel (26) zum Versetzen der Probennahmenadel (21) umfassen, die dafür ausgelegt sind, das Nachweisreagenz (R3) in die Messküvette (9) zu überführen.

14. Vorrichtung nach einem der vorhergehenden Ansprüche, wobei die zweiten Überführungsmittel ein Probennahmeventil (22) umfassen, das dafür ausgelegt ist, eine Probe in den ersten Zubereitungsmitteln (7) aufzunehmen.

15. Vorrichtung nach einem der vorhergehenden Ansprüche zur Analyse biologischer Parameter ausgehend von einer biologischen Probe, die eine Vollblutprobe (6) umfasst.

16. Vorrichtung nach Anspruch 15, die Mittel zur Messung eines Zellbestandteils (8, 50) umfasst, die dafür ausgelegt sind, mindestens eine Hämatokritmessung zu liefern.

17. Verfahren zur Analyse biologischer Parameter ausgehend von einer biologischen Probe (6), das die folgenden Schritte umfasst:

- Überführen mindestens eines Teils der biologischen Probe (6) durch erste Überführungsmittel (5, 20, 25) zu ersten Zubereitungsmitteln (7),

- Durchführen mindestens einer Verdünnung der biologischen Probe (6) mit mindestens einem Verdünnungsmittel und/oder einem Reagenz durch erste Zubereitungsmittel (7),
- Durchführen mindestens einer Verdünnung mit einem Nachweisreagenz (R3), das Partikel umfasst, die an der Oberfläche mit mindestens einem für mindestens einen Analyten von Interesse spezifischen Liganden funktionalisiert sind, an einer aus den ersten Zubereitungsmitteln (7) stammenden ersten Probe durch zweite Zubereitungsmittel (10, 11, 22, 23, 24),
- Nachweis mindestens eines Analyten von Interesse mit einer aus den zweiten Zubereitungsmitteln (10, 11, 22, 23, 24) stammenden Probe durch Immunnachweis-Messmittel (30, 31) durch Messen des Anteils an Aggregation von funktionalisierten Partikeln in einer Messküvette (9),

**dadurch gekennzeichnet, dass** es außerdem die folgenden Schritte umfasst:

- Aufnehmen der ersten Probe, die zuvor in den ersten Zubereitungsmitteln (7) verdünnt wurde, durch zweite Überführungsmittel (4, 21, 22, 26), die zumindest zum Teil von den ersten Überführungsmitteln (5, 20, 25) verschieden sind, und Überführen zu den zweiten Zubereitungsmitteln (10, 11, 22, 23, 24),
- Anlegen eines Magnetfelds in der Messküvette (9) derart, dass durch magnetische Wechselwirkung eine Beschleunigung der Aggregation der funktionalisierten Partikel, die kolloidale magnetische Partikel umfassen, hervorgerufen wird, und

einen Schritt des Erhaltens mindestens einer Messung des Zellvolumens in Bezug auf das Gesamtvolumen durch Mittel zum Messen eines Zellbestandteils (8) ausgehend von einer zweiten aus den ersten Zubereitungsmitteln (7) stammenden Probe (6).

18. Verfahren nach Anspruch 17, das außerdem einen Schritt des Erhaltens mindestens einer Messung des Zellvolumens in Bezug auf das Gesamtvolumen durch Mittel zum Messen eines Zellbestandteils (8, 50) ausgehend von der biologischen Probe (6) umfasst.

19. Verfahren nach einem der Ansprüche 17 oder 18, wobei der Nachweis mindestens einem Analyten von Interesse die folgenden Schritte umfasst:

- Einbringen einer Messlösung, die mindestens eine Probe und Nachweisreagenz (R3) umfasst, in die Messküvette (9),
- Messen eines ersten optische Intensität durch die Messküvette hindurch,
- Anlegen eines Magnetfelds in der Messküvette (9) während eines bestimmten Zeitraums, so dass die Aggregation der funktionalisierten Partikel unter der Einwirkung des Magnetfelds ermöglicht wird,

nach dem Abschalten des Magnetfelds Messen einer zweiten optischen Intensität, die für die restliche Aggregation der funktionalisierten Partikel aufgrund von Verbindungen zwischen Ligand und Analyt von Interesse repräsentativ ist,

- Berechnen einer Variation der optischen Dichte als Funktion des Verhältnisses der ersten und der zweiten optischen Intensität und
- Anwenden einer zuvor bestimmten Kalibrierfunktion zur Berechnung der Konzentration des Proteins von Interesse aus der Variation der optischen Dichte.

20. Verfahren nach Anspruch 19, wobei die biologische Probe zu mehr als x500 in der Messlösung verdünnt wird.

21. Verfahren nach einem der Ansprüche 17 bis 20 für die Analyse biologischer Parameter ausgehend von einer biologischen Probe (6), die eine Vollblutprobe umfasst.

22. Verfahren nach Anspruch 21, das zum Nachweis des Analyten von Interesse:

- ein lytisches Reagenz (R1), das Saponin umfasst,
- eine Pufferlösung (R2) zum Aufrechterhalten eines optimalen pH-Werts

einsetzt.

**23.** Verfahren nach einem der Ansprüche 21 oder 22, das Partikel einsetzt, die mit einem Liganden funktionalisiert sind, der dafür ausgelegt, einen Nachweis eines Analyten von Interesse, bei dem es sich um C-reaktives Protein (CRP) handelt, zu ermöglichen.

**Claims**

**1.** Device for analyzing biological parameters using a biological sample (6), comprising:

- first transferring means (5, 20, 25) capable of at least partially transferring said biological sample (6) to first preparing means (7),
- first preparing means (7) capable of carrying out at least one dilution of said biological sample (6) with at least one diluent and/or one reagent,
- second preparing means (10, 11, 22, 23, 24) capable of carrying out, on a first sample from the first preparing means (7), at least one dilution with an assay reagent (R3) comprising particles functionalized at the surface with at least one ligand specific for at least one analyte of interest,
- immunodetection measurement means (30, 31) capable of assaying, on a sample from the second preparing means (10, 11, 22, 23, 24), at least one analyte of interest by measuring the degree of aggregation of functionalized particles in a measuring cuvette (9),

**characterized in that** it also comprises:

- second transferring means (4, 21, 22, 26) at least partially separate from the first transferring means (5, 20, 25) and capable of taking said first sample diluted beforehand in the first preparing means (7), and of transferring it to the second preparing means (10, 11, 22, 23, 24),
- means for applying a magnetic field (28) in said measuring cuvette (9), capable of causing, by magnetic interaction, an acceleration of the aggregation of said functionalized particles, which comprise magnetic colloidal particles, and means for measuring a cell component (8) which are capable of providing, from a second sample from the first preparing means (7), at least one measurement of cell volume relative to the total volume.

**2.** Device of Claim 1, which also comprises means for measuring a cell component (8, 50) which are capable of providing, from the biological sample (6), at least one measurement of cell volume relative to the total volume.

**3.** Device of either of the preceding claims, wherein the functionalized particles comprise particles which have a core with a high iron oxide content surrounded by a shell made of a polymer material.

**4.** Device of one of the preceding claims, wherein the functionalized particles comprise particles having an essentially spherical shape with an average diameter of less than 1 micrometer.

**5.** Device of one of the preceding claims, wherein the immunodetection measurement means (2) comprise optical measurement means with at least one light source (30) and at least one detector (31) placed in proximity to the measuring cuvette (9), said cuvette having, at least at the level of said optical measurement means (30, 31), substantially transparent walls.

**6.** Device of Claim 5, wherein the immunodetection measurement means also comprise optical conditioning means capable of producing, from at least one light source (30), a collimated light beam (32) which passes through the measuring cuvette (9).

**7.** Device of either of Claims 5 and 6, which comprises at least one light source (30) capable of emitting in optical wavelengths of between 600 nanometers and 900 nanometers.

**8.** Device of one of the preceding claims, which also comprises an electromagnet (28) capable of producing a magnetic field in the measuring cuvette (9).

**9.** Device of one of the preceding claims, which also comprises means for regulating the temperature of the measuring cuvette (9).

**10.** Device of one of the preceding claims, which also comprises a storage container (11) regulated at an optimal temperature for storing the assay reagent (R3) .

11. Device of Claim 10, which also comprises ultrasonic agitation means (13) for placing and/or keeping the functionalized particles in suspension in the stored assay reagent (R3), comprising at least one of the following means: an external sonotrode coupled to the storage container, a sonotrode immersed in the assay reagent (R3).

12. Device of one of the preceding claims, wherein the first transferring means comprise a sampling needle (20) and means (25) for moving said sampling needle (20).

13. Device of one of the preceding claims, wherein the second transferring means comprise a sampling needle (21) and means (26) for moving said sampling needle (21) which are capable of transferring assay reagent (R3) into the measuring cuvette (9).

14. Device of one of the preceding claims, wherein the second transferring means comprise a sampling valve (22) capable of taking a sample from the first preparing means (7).

15. Device of one of the preceding claims, for analyzing biological parameters using a biological sample comprising a whole blood sample (6).

16. Device of Claim 15, which comprises means for measuring a cell component (8, 50) which are capable of providing at least one hematocrit measurement.

17. Method for analyzing biological parameters using a biological sample (6), comprising steps of:

- transferring, via first transferring means (5, 20, 25), at least one part of said biological sample (6) to first preparing means (7),
- carrying out, via first preparing means (7), at least one dilution of said biological sample (6) with at least one diluent and/or one reagent,
- carrying out, via second preparing means (10, 11, 22, 23, 24), on a first sample from the first preparing means (7), at least one dilution with an assay reagent (R3) comprising particles functionalized at the surface with at least one ligand specific for at least one analyte of interest,
- assaying, via immunodetection measurement means (30, 31), at least one analyte of interest with a sample from the second preparing means (10, 11, 22, 23, 24), by measuring, in a measuring cuvette (9), the degree of aggregation of functionalized particles,

**characterized in that** it also comprises steps of:

- taking, via second transferring means (4, 21, 22, 26), at least partially separate from the first transferring means (5, 20, 25), said first sample diluted beforehand in the first preparing means (7), and transferring to the second preparing means (10, 11, 22, 23, 24),
- applying a magnetic field in said measuring cuvette (9) so as to cause, by magnetic interaction, an acceleration of the aggregation of said functionalized particles, which comprise magnetic colloidal particles, and

a step of obtaining, via means for measuring a cell component (8), from a second sample from the first preparing means (7), at least one measurement of cell volume relative to the total volume.

18. Method of Claim 17, which also comprises a step of obtaining, via means for measuring a cell component (8, 50), from the biological sample (6), at least one measurement of cell volume relative to the total volume.

19. Method of either of Claims 17 and 18, wherein the assaying of at least one analyte of interest comprises steps of:

- introducing into the measuring cuvette (9) a measuring solution comprising at least one sample and assay reagent (R3),
- measuring a first optical intensity through the measuring cuvette,
- applying a magnetic field in the measuring cuvette (9) for a given period of time, so as to allow the aggregation of the functionalized particles under the effect of the magnetic field,
- after the magnetic field has been turned off, measuring a second optical intensity representative of the residual aggregation of the functionalized particles due to the couplings between ligand and analyte of interest,

- calculating a variation in optical density as a function of the ratio of said first and second optical intensities, and
- applying a calibration function determined beforehand so as to calculate the concentration of the protein of interest from the variation in optical density.

20. Method of Claim 19, wherein the biological sample is diluted to a degree greater than ×500 in the measuring solution.

21. Method of one of Claims 17 to 20, for analyzing biological parameters using a biological sample (6) which comprises a whole blood sample.

22. Method of Claim 21, which uses, for assaying the analyte of interest:

    - a lytic reagent (R1) comprising saponin,
    - a buffer solution (R2) capable of maintaining an optimal pH.

23. Method of either of Claims 21 and 22, which uses particles functionalized with a ligand enabling an analyte of interest, which is C-reactive protein (CRP), to be assayed.

Figure 1

Figure 2

Figure 3

Figure 4

Figure 5

Figure 6

Figure 7

Figure 8

**EP 2 810 042 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 6106778 A, Oku
- EP 1446666 A, Bibette
- WO 2009024710 A